# EUROPEAN PATENT SPECIFICATION

(11) **EP 2 207 795 B1**
(45) Date of publication and mention of the grant of the patent: **31.08.2011**
(21) Application number: 08805099.2
(22) Date of filing: 07.10.2008
(51) Int. Cl.: C07K 14/435

(54) **PROCESS FOR PRODUCING MATURE RECOMBINANT MITE GROUP I ALLERGEN**
VERFAHREN ZUR HERSTELLUNG VON REIFEM, REKOMBINANTEM MILBEN-I-ALLERGEN
PROCÉDÉ DE FABRICATION D'UN ALLERGÈNE DU GROUPE I DES ACARIENS RECOMBINANT MATURE

(30) Priority: 08.10.2007 DK 200701454; 09.10.2007 US 978690 P
(43) Date of publication of application: 21.07.2010
(73) Proprietor: ALK-Abelló A/S, 2970 Hørsholm (DK)
(72) Inventor: MONSALVE CLEMENTE, Rafael Ignacio, E-28220 Madrid (ES); HERNANDEZ, Domingo Barber, E-28027 Madrid (ES)
(74) Representative: Inspicos A/S
(86) International application number: PCT/EP2008/063379
(87) International publication number: WO 2009/047241

(56) References cited:
- WO-A-01/29078
- JACQUET A ET AL: "High-level expression of recombinant house dust mite allergen Der p 1 in Pichia pastoris" CLINICAL AND EXPERIMENTAL ALLERGY, vol. 32, no. 7, July 2002 (2002-07), pages 1048-1053, XP002485311 ISSN: 0954-7894 cited in the application

## Description

### TECHNICAL FIELD

The present invention relates to a process for producing mature recombinant Mite Group I allergen.

### BACKGROUND OF THE INVENTION

WO 01/29078, Example 2, describes the production of Derf 1 in Pichia Pastoris comprising cultivation in YPD medium at pH 6 to produce pro-Der f 1. The supernatant of the culture medium was recovered and diluted with buffer pH 4.5 and run in a SP-Sepharose column equilibrated with buffer pH 4.5. The protein was eluted with buffer pH 4.5 with a linear NaCl gradient in 20-25 column volumes. This procedure produced two mature forms of Der f 1, one of which has two additional amino acids at the N-terminal end com pared to the other. It is stated that P. pastoris is able to produce the pro-form and cleave it to produce the mature form. In Example 12 of WO 01/29078 it is speculated that pro-Der f 1 undergoes self-processing and an inactive mutant having a mutation in the active site was prepared. A corresponding mutant of Der p 1 was prepared. No method of expressing the mutants is indicated in Example 12.

Yasuhara et al. (Clinical and Experimental Allergy, 2001, Volume 31, pages 116-124 ) describes a process for producing rDer f 1, wherein expression of the proform of wild-type Der f 1 is obtained in a step, wherein Pichia Pastoris is grown in BMMY medium at pH 6.0 for 48-72 hours, after which the supernatant was harvested by centifugation. In vitro activation was effected autocatalytically by dialysis against 100 mM acetate buffer (pH 4.0) at 4 C for 48 hours.

Jacquet et al. (Clin Exp Allergy 2002, 32_1048-1053) discloses the production of Der p 1 comprising growing P. pastoris containing plasmids with cDNA encoding proDer p 1 in buffered minimal glucose (BMG) medium. Expression was induced by daily addition of methanol 0.5 %. The supernatant was collected by centrifugation. The collected proDer p 1 was purified on a column and by ultrafiltration. The purified proDer p 1 was subjected to maturation by autocatalytic processing of Der p 1 by dialysis against 100 mM sodium acetate buffer pH 4.

De Halleux et al. (J Allergy Clin Immunol, 2006) discloses the production of wildtype rproDer p 1 and a N52Q unglycosylated mutant of rproder p 1. Expression was carried out in Pichia pastoris X-33 using pPICZaC as expression vector. The culture was grown at 30 °C in a specified fermentation medium. The culture was then induced with methanol for 3 days at 30. The pH of the medium was adjusted to 6.0 during growth and induction. The cultivation mixture resulting from the fermentation was centrifuged and the supernatant was dialysed against 25 mmol/L sodium acetate buffer pH 4.5 and kept at 4.5 C for about 3 to 4 days for automaturation of pro-Der p 1 and pro-Der p 1 N52Q into mature allergens. Then purification was performed by cationic exchange on SP-Sepharose and additional steps.

The object of the present invention is to provide an improved process for producing recombinant mature Mite Group I allergen.

### SUMMARY OF THE INVENTION

This object is achieved with the present invention, which relates to a process for producing mature recombinant Mite Group I allergen comprising the steps of
a) providing an allergen expression system in the form of a host cell selected from the group of yeasts containing a vector comprising cDNA encoding pro-allergen,
b) cultivating the allergen expression system at a cultivation pH of between 5.5 and 7.5 for a cultivation period to express pro-allergen to obtain a cultivation mixture containing pro-allergen,
c) adjusting the pH of the cultivation mixture to a maturation pH of between 3.5 and 5.0,
d) maintaining the cultivation mixture at the maturation pH for a maturation period to convert the pro-allergen into mature allergen to obtain a product mixture, and
e) subjecting the product mixture to a purification method to isolate mature allergen from the product mixture.

The present invention is based on the experimental finding that it is possible to carry out maturation of pro-allergen to obtain mature allergen in the cultivation mixture wherein expression has been carried out without any prior separation or purification of the product from the cultivation mixture. Such a process involves a number of advantages compared to the prior art processes. Firstly, the process of the invention is simplified in that it allows the expression and the maturation to be carried out in the same reactor, and hence the need for a separate maturation step is eliminated. Secondly, the maturation may be carried out immediately after the expression has been completed without any intervening purification steps, which makes it possible to control the maturation process more effectively, in particular to avoid uncontrolled maturation to take place under the purification steps.

A third advantage of the process of the invention is that it allows a reduction of the period of maturation, which is important to minimize degradation of both pro-allergen and mature allergen, and hence to increase the yield.

A fourth advantage is that the expression and secretion are allowed to continue during the maturation period, which increases the yield and/or allows a reduction of the period of expression.

A fifth advantage is that the purification steps are simplified, since only the mature form of the allergen is being isolated, whereas in the prior art processes the expression step will usually result in fermentation product containing a mixture of pro-allergen and mature allergen, which makes additional purification steps necessary.

### SHORT DESCRIPTIONOF THE FIGURES

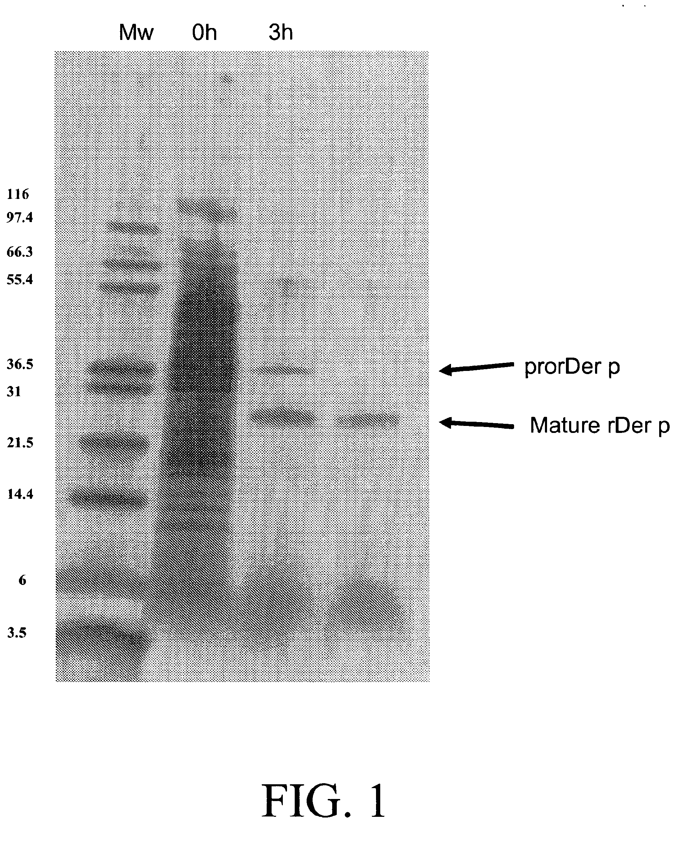

Fig. 1 shows an SDS-PAGE of cultivation mixture samples taken during the maturation period at times 0, 3 and 18 hours.

### DETAILED DESCRIPTION OF THE INVENTION

### Mite Group I allergen

The Mite Group I allergen produced by the process of the invention may be any known Mite Group I allergen of the order Astigmata, in particular an allergen selected from the group consisting of the superfamilies Acaroidea, Glycyphagoidea and Analgoidea, more particularly selected from the group consisting of the families Acaridae, Glycyphagidae, Echimyopodidae and Pyroglyphidae, more particularly selected from the group consisting of the genus Acarus, Blomia, Dermatophagoides, Euroglyphus, Glycyphagus, Lepidoglyphus and Tyrophagus. In a particular embodiment of the invention the Mite Group I allergen is selected from the group consisting of Aca s 1, Blo t1, Der f 1, Der p 1, Eur m 1, Gly d 1, Lep d 1 and Tyr p 1. Particularly, the Mite Group I allergen is selected from the group consisting of Blo t1, Der f 1, Der p 1 and Eur m 1. More particularly, the Mite Group I allergen is Der p 1.

Furthermore, the Mite Group I allergen produced by the process of the invention may be a naturally occurring isoform of the above-mentioned allergens, as well as a mutated variant of the above-mentioned allergens, e.g. a mutated variant prepared by recombinant techniques. Mutations may e.g. be made to obtain hypoallergenic variants of the wildtype allergens, to make the allergens more stable and/or easier to express and purify and to change the glycosylation pattern of the allergen, e.g. by substituting an amino acid carrying a glycan side chain.

### Allergen expression system

The host cell may be any yeast suitable for this purpose, in particular yeasts of the order saccharomycetales, more particularly yeasts selected from the group consisting of the genus Pichia, Saccharomyces and Candida. In a particular embodiment of the invention, the host cell is of the family saccharomycetaceae, more particularly selected from the group consisting of the genus Pichia and Saccharomyces, and most particularly selected from the group consisting of the species Pichia pastoris and Saccharomyces cerevisiae.

The vector may be any conventional vector used as a carrier of the DNA encoding the pro-allergen, and which may be introduced into the host cell and expressed therein. In particular, the vector may be a plasmid or a phage. Examples of suitable vectors are the E.coli/P. Pastoris shuttle vector pGAPZαA, pPICZαA, pPICZαB, pPICZαC, pPIC9K and pHIL-S1.

In a particular embodiment of the invention, the vector includes an constitutive promoter, such as that of the GAP gene encoding glyceraldehydes-3-phosphate dehydrogenase. In another particular embodiment of the invention, the vector includes a methanol-inducible promoter, such as the AOX promoter. In a particular embodiment of the invention, the vector includes a signal sequence for secretion, such as the Saccharomyces cerevisiae α factor and PHO1.

In a particular embodiment of the invention, the yeast is Pichia pastoris and the vector is pGAPZaA.

In a particular embodiment of the invention, the amino acid sequence of the allergen is modified so as to include a number of histidine residues at the C-terminal end of the molecule. Such a sequence of histidine residues, which is usually referred to as a HIS-tag, form a metal binding site for use in affinity chromatography for simplifying purification of the recombinant molecule from the fermentation liquid mixture.

### Expression of pro-allergen

The allergen expression system is cultivated at a cultivation pH of between 5.5 and 7.5 for a cultivation period to express pro-allergen to obtain a cultivation mixture containing pro-allergen. In particular, the cultivation pH is between 5.8 and 7.2, more preferably between 6.0 and 7.0, more preferably between 6.2 and 6.8.

In a particular embodiment of the invention, the cultivation period is from 12 hours to 144 hours, preferably from 24 hours to 120 hours, more preferably from 48 hours to 96 hours.

### Maturation of proDer p1 into mature Der p1

The pH of the cultivation mixture is adjusted to a maturation pH of between 3.5 and 5.0. In a particular embodiment of the invention, the maturation pH is between 3.7 and 4.8, more particularly between 3.9 and 4.6, and most particularly between 4.0 and 4.5.

In another particular embodiment of the invention, the maturation period is from 8 hours to 30 hours, particularly from 12 hours to 26 hours, more particularly from 14 hours to 24 hours, more particularly from 16 hours to 22 hours and most particularly from 17 hours to 21 hours.

### Purification method

Any conventional method for separating a protein product from a fermentation liquid mixture may be employed in the process of the invention as purification method to isolate mature allergen from the product mixture resulting from the maturation.

Examples of suitable purification methods are centrifugation, filtration, ultrafiltration, diafiltration, dialysis, gelfiltration, precipitation, affinity chromatography and combinations of these methods.

### DEFINITIONS

In connection with the present invention the following definitions are used:

The term "pro-allergen" means the pro-form of an allergen molecule, i.e. an allergen molecule containing both the mature part and the pro-peptide of the molecule.

### EXAMPLES

### Example 1: Production of recombinant Der p1

This example provides a method for producing mature recombinant Der p 1 after expression of the pro-Der p 1 form in the methylotropic yeast Pichia pastoris. The maturation of the proform is carried out in the fermentation vessels, by adjusting pH to 4.0 during a definite period of time so that all the pro-Der p 1 is processed to a mature Der p 1 molecule that can be subsequently purified.

### Construction of rproDer p 1 variants:

A partially codon optimised rproderp1 wild type (wt) (from now termed rproderp1) cDNA containing the complete coding region for rproDer p 1 wt (from now termed rproder p 1) with the addition of a 10 aa C-terminal His tag was constructed by PCR. The encoded rproder p 1 protein is equivalent to the proDer p 1 region (aa 19-320) of UniProt accession number P08176 with the exception V204A (aa numbering throughout this Example is from the first aa in proDer p 1), which is a naturally occurring variant. A forward primer and a reverse primer was used to create a 964 bp fragment with Xhol and Xbal restriction sites introduced near the 5' and 3' end, respectively. This rproderp1 gene was cloned into pCR4-TOPO, transformed into TOP10 (Invitrogen) and the sequence confirmed. Recloning of the rproder p 1 gene into the E. coli/P. pastoris shuttle vector pGAPZaA was subsequently done by directional cloning with the restriction enzymes Xhol and Xbal. This resulted in plasmid rproderp1 with the rproder p 1 encoded region downstream of the Saccharomyces cerevisae α factor with a Kex2 cleavage site between the two proteins facilitating secretion of the recombinant protein.

The plasmid was initially transformed into E. coli TOP10 cells.

Construction of site specific mutation N132D was done by the overlap extension method. Briefly, a forward primer 1 was used together with a reverse primer 7 in one PCR-reaction, a reverse primer 3 was used together with a forward primer 6 in another PCR reaction and a reverse primer 2 was used together with a forward primer 8 in a third PCR reaction, all with proderp1 as template. The full fragment containing the mutation was then created by using the three resulting PCR-products together with primer 1 and 2 in a final PCR-reaction followed by directional cloning of the 957 bp XhoI - Xbal-fragment into pGAPZaA as described for proderp1 creating plasmid rproderp1-N132D (from now termed rproderp1-N).

### Expression and purification of rproDer p 1 variants:

pGAPZaA-rproderp1-N was transformed into P. pastoris X33 wt strain (Invitrogen) according to the manufacturers protocol. Clones were restreaked onto YPDZ-agar plates and checked for expression. The P. pastoris X33::rproder p 1-N clones expressing rproder p 1-N132E (from now termed rproder p 1-N) were grown as described in the protocol supplied by the manufacturer (Invitrogen), briefly: 5 ml YPDZ medium (YPD + 100µg/ml Zeocin) was inoculated with cells from one single colony and grown over night in a shaker at 220 rpm at 30°C. 2 ml of this culture was used for inoculating 400 ml of fresh YPD (yeast extract/peptone/dextrose) medium that was incubated overnight before using it for inoculating 3.6 I of YPD medium to be used for the fermentation.

Fermentation is carried out in a B. Braun-Biotech International system (BIOSTAT B-DCU model), in vessels of 5-L maximum capacity, with the following basic fermentation conditions:
- temperature at 30°C
- stirring speed of 800rpm
- airflow supply of 0.8 volume per minute
- pH controlled initially at pH 6.5, by means of automatic addition of 30% NH₄OH or 5% H₂SO₄.

Fermentation is carried out in a complex medium named YPD, being its composition 1% yeast extract, 2% soy peptone, and 2% dextrose. Cell density of the growing culture is controlled by optical density measurements at 600nm. Dissolved oxygen, measured by means of a pO₂ electrode, is controlled in order to follow cellular division (low pO₂ levels indicate high oxygen consumption due to high cellular activity, and increasing pO₂ levels indicate lower cellular activity or cellular division due to, for example, depletion of carbon source). If addition of carbon source is necessary, a solution containing 5% dextrose, 13.4% Yeast Nitrogen Base (YNB), and 0.02% Biotin is added to the ongoing culture.

### Adjustment of pH and maturation of proder p1 into mature Der p 1:

After 48h of fermentation, with a controlled pH of 6.5, the recombinant product expressed is r-pro-Der p 1, as it can be confirmed by SDS-PAGE. In order for the maturation process to start, the pH is diminished to pH 4.0 and maintained at that level, while other fermentation conditions are kept constant. After 18 hours at acidic pH, only mature r-Der p 1 is observed as recombinant product, cf. Fig. 1. At the moment of proceeding to isolating mature r-Der p 1, in order to avoid further proteases activity, pH is raised to pH 8.0, and the culture is harvested.

### Purification method:

The culture fluid that contains the secreted recombinant product is clarified and separated from the yeast cells by centrifugation and subsequent filtration. (NH₄)₂SO₄ (ammonium sulphate) precipitation is carried out in two steps, first reaching a concentration of 1.8M (NH₄)₂SO₄, followed by separation of precipitated and soluble products, and in a second step reaching a 3.2 M salt concentration of the supernatant obtained after the first step. This high salt concentration induces selective precipitation of the recombinant product from the supernatant resulting from the first step, which contains a number of protein contaminants, and hence allows purification of the recombinant product. All these steps are carried out at 4 °C. After centrifugation, pelleted precipitated material is separated from components soluble at high salt concentrations, and the pellets are stored at -20°C.

Further purification of the recombinant product (mature r-Der p 1) is achieved by affinity chromatography, on BD-TALON metal affinity (BD Biosciences), of the dialyzed sample. The recombinant product is specifically bound to the column through the Histidine-tag, and elutes with by addition to the chromatography buffer (50mM sodium phosphate, 300mM NaCl, pH 8.0) of Imidazole to a final concentration of 300mM. Chromatographic steps are carried out on an AKTAexplorer platform, at 4°C. The eluted peak is dialyzed to buffer saline and the sample is kept frozen.

### Conclusions:

As will appear from the above the present Example shows that it is possible to carry out the cultivation to express the pro-allergen and its maturation in the same reactor, i.e. without carrying out any steps of separating the pro-allergen from the remaining components of the cultivation mixture prior to maturation. Also, this Example shows it is possible to carry out the maturation in a relatively short period of time (18 hours).

## Claims

1. A process for producing mature recombinant Mite Group I allergen comprising the steps of
a) providing an allergen expression system in the form of a host cell selected from the group of yeasts containing a vector comprising cDNA encoding pro-allergen,
b) cultivating the allergen expression system at a cultivation pH of between 5.5 and 7.5 for a cultivation period to express pro-allergen to obtain a cultivation mixture containing pro-allergen,
c) adjusting the pH of the cultivation mixture to a maturation pH of between 3.5 and 5.0,
d) maintaining the cultivation mixture at the maturation pH for a maturation period to convert the pro-allergen into mature allergen to obtain a product mixture, and
e) subjecting the product mixture to a purification method to isolate mature allergen from the product mixture.

2. A process according to claim 1, wherein the yeast is selected from the group consisting of the genus Pichia, Saccharomyces and Candida.

3. A process according to claim 2, wherein the yeast is Pichia pastoris.

4. A process according to any of claims 1-3, wherein the vector is selected from the group consisting of plasmids and phages.

5. A process according to claim 4, wherein the vector is a plasmid.

6. A process according to claim 5, wherein the vector is selected from the group consisting of pGAPZaA, pPICZaA, pPICZaB, pPICZaC, pPIC9K and pHIL-S1.

7. A process according to claim 6, wherein the vector is pGAPZaA.

8. A process according to any of claims 1-7, wherein the cultivation period is from 12 hours to 144 hours, preferably from 24 hours to 120 hours, more preferably from 48 hours to 96 hours.

9. A process according to any of claims 1-8, wherein the maturation period is from 8 hours to 30 hours, preferably from 12 hours to 26 hours, more preferably from 14 hours to 24 hours, more preferably from 16 hours to 22 hours and most preferably from 17 hours to 21 hours.

10. A process according to any of claims 1-9, wherein the cultivation pH is between 5.8 and 7.2, more preferably between 6.0 and 7.0, more preferably between 6.2 and 6.8.

11. A process according to any of claims 1-10, wherein the maturation pH is between 3.7and 4.8, more preferably between 3.9 and 4.6, and most preferably between 4.0 and 4.5.

12. A process according to any of claims 1-11, wherein the Mite Group I allergen is selected from the group consisting of Aca s 1, Blo t1, Der f 1, Der p 1, Eur m 1, Gly d 1, Lep d 1 and Tyr p 1.

13. A process according to claim 12, wherein the Mite Group I allergen is selected from the group consisting of Blo t1, Der f 1, Der p 1 and Eur m 1.

14. A process according to claim 13, wherein the Mite Group I allergen is Der p1.

## Patentansprüche

1. Verfahren zur Herstellung eines gereiften rekombinanten Milbenallergens der Gruppe I, wobei das Verfahren folgende Schritte umfaßt:
a) Bereitstellen eines Expressionssystems für das Allergen in Form einer Wirtszelle, die ausgewählt ist aus der Gruppe von Hefen, die einen Vektor enthalten, der eine Pro-Allergen kodierende cDNA umfaßt,
b) Kultivieren des Expressionssystems für das Allergen bei einem pH-Wert für das Kultivieren zwischen 5.5 und 7.5 während einer zur Expression des Pro-Allergens erforderlichen Kultivierungszeit, um eine Mischkultur zu erhalten, die das Pro-Allergen aufweist,
c) Einstellen des pH-Wertes der Mischkultur auf einen pH-Wert für die Reifung zwischen 3.5 und 5.0,
d) Halten der Mischkultur bei dem pH-Wert während einer zur Umwandlung des Pro-Allergens in das reife Allergen erforderlichen Reifezeit, um eine Produktmischung zu erhalten, und
e) Behandeln der Produkt-Mischung in einem Reinigungsverfahren, um gereiftes Allergen aus der Produkt-Mischung zu erhalten.

2. Verfahren nach Anspruch 1, wobei die Hefe ausgewählt ist aus der Gruppe bestehend aus Genus Pichia, Saccharomyces und Candida.

3. Verfahren nach Anspruch 2, wobei die Hefe Pichia pastoris ist.

4. Verfahren nach einem der Ansprüche 1-3, wobei der Vektor ausgewählt ist aus der Gruppe bestehend aus Plasmiden und Phagen.

5. Verfahren nach Anspruch 4, wobei der Vektor ein Plasmid ist.

6. Verfahren nach Anspruch 5, wobei der Vektor ausgewählt ist aus der Gruppe bestehend aus pGAPZαA, pPICZαA, pPICZαB, pPICZαC, pPIC9K und pHIL-S1.

7. Verfahren nach Anspruch 6, wobei der Vektor pGAPZaA ist.

8. Verfahren nach einem der Ansprüche 1-7, wobei die Kultivierungszeit 12 bis 144 Stunden, vorzugsweise 24 bis 120 Stunden, besonders bevorzugt 48 bis 96 Stunden beträgt.

9. Verfahren nach einem der Ansprüche 1-8, wobei die Reifezeit 8 bis 30 Stunden, vorzugsweise 12 bis 26 Stunden, besonders bevorzugt 14 bis 24 Stunden, besonders bevorzugt 16 bis 22 Stunden und am meisten bevorzugt 17 bis 21 beträgt.

10. Verfahren nach einem der Ansprüche 1-9, wobei der pH-Wert für das Kultivieren zwischen 5.8 und 7.2, besonders bevorzugt zwischen 6.0 und 7.0, besonders bevorzugt zwischen 6.2 und 6.8 beträgt.

11. Verfahren nach einem der Ansprüche 1-10, wobei der pH-Wert für das Reifen zwischen 3.7 und 4.8, besonders bevorzugt zwischen 3.9 und 4.6 und am meisten bevorzugt zwischen 4.0 und 4.5 beträgt.

12. Verfahren nach einem der Ansprüche 1-11, wobei das Milbenallergen der Gruppe I ausgewählt ist aus der Gruppe bestehend aus Aca s 1, Blo t1, Der f 1, Der p 1, Eur m 1, Gly d 1, Lep d 1 und Tyr p 1.

13. Verfahren nach Anspruch 12, wobei das Milbenallergen der Gruppe I ausgewählt ist aus der Gruppe bestehend aus Blo t 1, Der f 1, Der p 1 und Eur m1.

14. Verfahren nach Anspruch 13, wobei das Milbenallergen der Gruppe I Der p 1 ist.

## Revendications

1. Procédé de production d'un allergène recombinant mature du groupe I des acariens, qui comprend les étapes consistant à :
a) fournir un système d'expression d'allergène sous la forme d'une cellule hôte choisie dans le groupe constitué par les levures contenant un vecteur comprenant un ADNc codant pour un pro-allergène,
b) cultiver le système d'expression d'allergène à un pH de culture compris entre 5,5 et 7,5 pendant une période de culture pour exprimer un pro-allergène pour obtenir un mélange de culture contenant un pro-allergène,
c) ajuster le pH du mélange de culture à un pH de maturation compris entre 3,5 et 5,0,
d) maintenir le mélange de culture au pH de maturation pendant une période de maturation pour convertir le pro-allergène en allergène mature pour obtenir un mélange de produits, et
e) soumettre le mélange de produits à un procédé de purification pour isoler l'allergène mature du mélange de produits.

2. Procédé selon la revendication 1, dans lequel la levure est choisie dans le groupe constitué par les genres Pichia, Saccharomyces et Candida.

3. Procédé selon la revendication 2, dans lequel la levure est Pichia Pastoris.

4. Procédé selon l'une quelconque des revendications 1 à 3, dans lequel le vecteur est choisi dans le groupe constitué par les plasmides et les phages.

5. Procédé selon la revendication 4, dans lequel le vecteur étant un plasmide.

6. Procédé selon la revendication 5, dans lequel le vecteur est choisi dans le groupe constitué par pGAP2αA, pPIC2αA, pPIC2αB, pPIC2αC, pPIC9K et pHIL-S1.

7. Procédé selon la revendication 6, dans lequel le vecteur étant pGAP2αA.

8. Procédé selon l'une quelconque des revendications 1 à 7, dans lequel la période de culture dure de 12 heures à 144 heures, de préférence de 24 heures à 120 heures, de manière davantage préférée de 48 heures à 96 heures.

9. Procédé selon l'une quelconque des revendications 1 à 8, dans lequel la période de maturation dure de 8 heures à 30 heures, de préférence de 12 heures à 26 heures, de manière davantage préférée de 14 heures à 24 heures, de manière encore davantage préférée de 16 heures à 22 heures et idéalement de 17 heures à 21 heures.

10. Procédé selon l'une quelconque des revendications 1 à 9, dans lequel le pH de culture est compris entre 5,8 et 7,2, de préférence entre 6,0 et 7,0, et de manière davantage préférée entre 6,2 et 6,8.

11. Procédé selon l'une quelconque des revendications 1 à 10, dans lequel le pH de maturation est compris entre 3,7 et 4,8, de préférence entre 3,9 et 4,6, et de manière davantage préférée entre 4,0 et 4, 5.

12. Procédé selon l'une quelconque des revendications 1 à 11, dans lequel l'allergène du groupe I des acariens est choisi dans le groupe constitué par Aca s 1, Blo t 1, Der f 1, Der p 1, Eur m 1, Gly d 1, Lep d 1, Tyr p 1.

13. Procédé selon la revendication 12, dans lequel l'allergène du groupe I des acariens est choisi dans le groupe constitué par Blo t 1, Der f 1, Der p 1 et Eur m 1.

14. Procédé selon la revendication 13, dans lequel l'allergène du groupe I des acariens est Der p 1.
